# EUROPEAN PATENT SPECIFICATION

(11) **EP 0 486 510 B1**
(45) Date of publication and mention of the grant of the patent: **14.02.1996**
(21) Application number: 90910659.3
(22) Date of filing: 29.06.1990
(51) Int. Cl.: C07C 233/65, C07C 233/69, A01N 37/24, A23L 1/035

(54) **CYCLIC AMIDOCARBOXY SURFACTANTS, SYNTHESIS AND USE THEREOF**
ZYKLISCHE AMIDOKARBOXYOBERFLÄCHENAKTIVE VERBINDUNGEN, HERSTELLUNG UND DEREN VERWENDUNG
TENSIO-ACTIFS AMIDOCARBOXY CYCLIQUES, LEUR SYNTHESE ET LEUR EMPLOI

(30) Priority: 08.08.1989 US 391187; 27.06.1990 US 542780
(43) Date of publication of application: 27.05.1992
(60) Divisional application: 95201081.7
(73) Proprietor: STEPAN COMPANY, Northfield, IL 60093 (US)
(72) Inventor: GOZE, Jean, M., Mundelein, IL 60060 (US); BERNHARDT, Randal, J., Lindenhurst, IL 60046 (US); HARTLAGE, James, A., Northbrook, IL 60062 (US); SMITH, Norman, R., Lake Forest, IL 60045 (US); LIETZ, Dennis, E., Deerfield, IL 60015 (US); KAY, James, W., Durham, PA 18039 (US); BREITBEIL, Fred, W., Arlington Heights, IL 60005 (US); SAJIC, Branco, Chicago, IL 60625 (US); ROCKWELL, Ned, Miles, Lake Bluff, IL 60044 (US); McCONNELL, Nina, Montserrat, Winnetka, IL 60093 (US); MOHRING, William, Richard, Glenview, IL 60025 (US)
(74) Representative: Guerre, Dominique
(86) International application number: EP9001044
(87) International publication number: WO9101970

(56) References cited:
- EP-A- 0 031 924
- DE-A- 3 222 996
- DE-A- 3 701 719
- FR-A- 805 339
- US-A- 2 101 323
- US-A- 2 378 442
- US-A- 2 378 443
- US-A- 2 507 055
- US-A- 2 554 274
- US-A- 2 582 670
- US-A- 3 095 286
- US-A- 4 402 708
- Chemical Abstracts, volume 99, no. 6, 8 August 1983 (Columbus, Ohio, US), G.A.Shlykova: "Flotation properties of N-alkylmonoamides of phtalic acid", see page119, abstract 40544f, & Fiz.-Tekh. Tekhnol. Probl. Razrab. Obogashch. Tverd.Polezn. Iskop., (Mater. Konf. Molodykh. Uch.), 11th 1981 (Pub. 1982) 169-76
- Research Disclosure, volume 170, June 1978, (Havant, GB), "Viscosifiers for oil base drilling nuds", pages 37-38, see abstract no. 17052

## Description

### Oil and Water Emulsions

The invention relates to oil and water emulsions which comprise particular emulsifiers and which show improved stability.

A wide variety of surface active (surfactant) compounds are known and widely used. Further, certain relatively specific phthalamate derivatives are at least nominally disclosed in academic and patent literature. Some specific phthalamate derivatives have been suggested as being useful in plant growth regulator formulations, insect repellant formulations, bactericidal, fungicidal, herbicidal formulations, additives for improving low temperature flow characteristics of petroleum distillate fuels, solvent extraction formulations for certain heavy metal ions, catalyst systems for polyurethane foam formulations, additives for thermal recording materials, thickeners for silicone grease and oil-based drilling muds, additives for water-insensitive coatings, plasticizers, etc. For example, U.S. Patent 2,101,323 provides a scant disclosure of certain monoamides of particular dicarboxylic acids including cyclic amidocarboxy compounds and various phthalamate derivatives, solvent-based synthesis routes and a recitation of potential uses therefor. Somewhat similarly, U.S. Patent 2,467,192 provides a disclosure of certain ammonium salts of "amic" acids, including various phthalamate derivatives, synthesis thereof and a use therefor as a plasticizer which may involve forming water-insensitive surfaces or films. Numerous other, relatively remote, disclosures involving phthalamic acids or phthalamate derivatives include the following U.S. Patents: 2,554,249 (additive for insecticidal compositions); 2,582,670 (additive for vulcanization activators); 2,742,496 (additive for rust and corrosion inhibitor formulations); 3,095,286 (additive to screen-clogging prevention and rust inhibition formulations); 4,211,534 (additive for improving low temperature flow characteristics of petroleum fuel oils); 4,478,958 (additive to catalyst systems for polyurethane foam formulations); etc.

In addition, so-called fast-breaking personal-care compositions involving vinyl polymers are disclosed in European Patent Application Number 0268164 A2. Further, an oil-in-water hair/skin conditioning composition containing certain water-insoluble, unctuous oleaginous materials and a water-dispersable hydrated polyvalent metal salt (i.e. Al), adjusted to a relatively low pH so as to invert to a water-in-oil emulsion when rubbed onto hair/skin and provide a moisturizing or protective barrier is disclosed in U.S. Patent 4,551,330.

Further, U.S. Patents 3,928,432 and 4,544,726 disclose certain monomeric latex polymerization surfactants, which during polymerization are chemically incorporated into the polymers so as to prevent migration thereof and thereby reduce water-sensitivity of the resulting polymeric mass.

U.S. Patent 4,741,855 describes shampoo compositions which comprise a synthetic surfactant, an insoluble, non-volatile silicone, a suspending agent, and water. The described suspending agents include long chain esters of ethylene glycol, esters of long chain fatty amine oxides and many others. There appear to be several key conditioning components in these compositions, including an insoluble, non-volatile, silicone, a suspending agent and a quaternary ammonium compound. The quaternary ammonium compounds, disclosed in U.S. Patent no. 4,741,855 as ingredients in a shampoo composition are di(hydrogenated tallow) dimethyl ammonium chloride and cetyltrimethyl ammonium chloride.

The use of silicone material in shampoos has been described in a number of different publications. The manufacture of such compositions is extremely complicated and requires specialized mixing equipment, high shear pumps, a heat exchanger, several manufacturing tanks, etc.

Quaternary ammonium compounds derived from fatty acid amines such as tallow amine and di-tallow amine have been used as conditioners, surfactants and thickeners or emulsifiers in various shampoo and hair care products. For example, European Patent Application No. 0067635A2 discloses conditioning shampoos containing quaternary ammonium compounds of the formula: 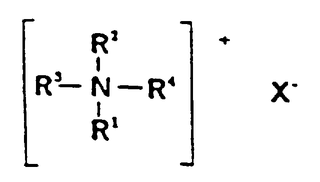 wherein the R¹ and R groups contain an average of from about 16 to 22 carbon atoms, most preferably from about 16 to about 18 carbon atoms, R³ and R⁴ are C₁ to C₄ alkyl or hydroxyalkyl groups, and X is any compatible anion, particularly one selected from the group consisting of halide, hydroxide, methylsulfate, or acetate anions.

The shampoo compositions of that patent application also contain acyl derivatives which are long chain amides, alkanolamides, esters of ethylene glycol and glycerine, esters of carboxylic acids, esters of thiodicarboxylic acids, and mixtures of these derivatives. The shampoo compositions of that patent application also contain surfactant which are represented by the formula: 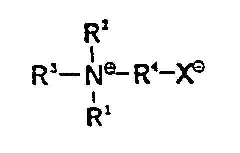 wherein R¹ is a long chain alkyl radical having from about 10 to about 18 carbon atoms or an amido radical represented by the formula:

R⁵-CONH (CH₂)₃

wherein R⁵ is a long chain alkyl radical, R and R³ are each alkyl radicals having from about 1 to about 3 carbon atoms. R⁴ is an alkylene or hydroxy alkylene radical having from about 1 to about 4 carbon atoms, and X is a carboxylate radical.

European Patent Application No. 0 152 194 A2 discloses shampoo compositions containing quaternary ammonium salts of the formula: 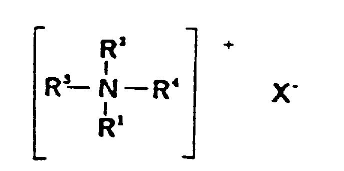 wherein R₁ is hydrogen, or an aliphatic group of from 1 to 22 carbon atoms, or an aromatic, aryl or alkaryl group having 6 to 20 carbon atoms; R₂ is an aliphatic group having from 12 to 22 carbon atoms; R₃ and R₄ are each alkyl groups having from 1 to 3 carbon atoms; and X is an anion selected from halogen, acetate, phosphate, nitrate and methyl sulfate radicals.

U.K. Patent Application No. GB 2196979 A provides hair care compositions comprising compounds of the formula: 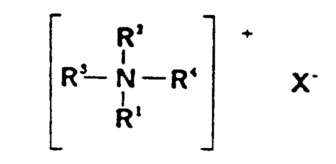 wherein R₁ and R₂ are aliphatic groups containing from about 12 to about 22 carbon atoms, R₃ and R₄ are hydrogen or short chain alkyl groups containing from about 1 to about 4 carbon atoms and X is anion selected from halogen, acetate, phosphate, nitrate and alkyl sulfate radicals.

U.K. Patent Application GB 2124647 A teaches quaternary ammonium compounds useful in shampoo compositions. The ammonium compounds have the formula: 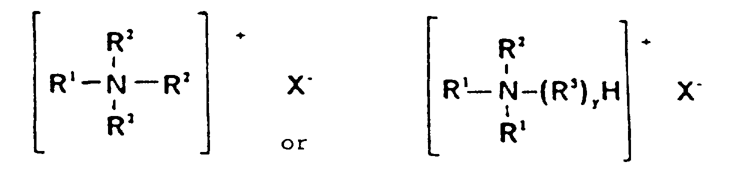 wherein R₁ is an aliphatic alkyl group containing an average of from about 16 to 22 carbon atoms, most preferably from about 16 to about 18 carbon atoms, the R₂ groups are C₁ to C₄ alkyl or hydroxyalkyl groups, the R₃ groups are alkylene oxide groups, preferably propylene oxide, where y is 1-4 and X is any compatible anion, particularly one selected from the group consisting of halide, hydroxide, methylsulfate, or acetate anions.

European Patent Application No. 0294 894 discloses conditioning agents for delivery from shampoos comprising compounds of the formula: 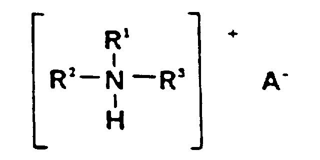 wherein R₁ and R₂ can independently be C₁₆ to C₂₀ alkyl or alkenyl and R³ is H or CH₃, and A is an anionic surfactant selected from the group consisting of alkyl sulfonates, aryl sulfonates, alkylaryl sulfonates, alkyl sulfates, alkyl ethoxylated sulfates, dialkyl sulfosuccinates, ethoxylated alkyl sulfonates, alkyl oxybenzene sulfonates, acyl isethionates, acyl alkyl taurates, olefin sulfonates and paraffin sulfonates.

A wide variety of surface active (surfactant) compounds are known and widely used. Further, certain relatively specific phthalamate derivatives are at least nominally disclosed in academic and patent literature. Some specific phthalamate derivatives have been suggested as being useful in plant growth regulator formulations, insect repellant formulations, bactericidal, fungicidal, herbicidal formulations, additives for improving low temperature flow characteristics of petroleum distillate fuels, solvent extraction formulations for certain heavy metal ions, catalyst systems for polyurethane foam formulations, additives for thermal recording materials, thickeners for silicone grease and oil-based drilling muds, additives for water-insensitive coatings, plasticizers, etc. Phthalamic acids or phthalamate derivatives have been used as additives for insecticidal compositions; additives for vulcanization activators; additives for rust and corrosion inhibitor formulations; additives to screen-clogging prevention and rust inhibition formulations; additives for improving low temperature flow characteristics of petroleum fuel oils; additives to catalyst systems for polyurethane foam formulations.

Ammonium phthalamates have been used as additives in fuel oil compositions, blending agents for grease, lubricating oil additives, and thickening agents for lubricating oil compositions. These ammonium phthalamates have the formula 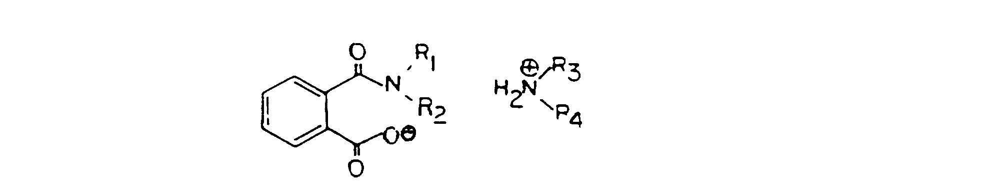
wherein R₁, R₂, R₃ and R₄ are the C₁₆-C₄₀, preferably C₁₆-C₂₄ straight chain alkyl groups of secondary amine, and may be the same or different.

Tallow is a fatty acid byproduct of the meat-packing industry obtained by rendering the body fat from cattle and sheep. Tallows from different sources vary in free fatty acid content. The fatty acids normally found in tallow are myristic acid, palmitic acid, stearic acid, oleic acid, and linoleic acid.

Several methods are known for the preparation of tallow amines, but the most common method in industry is the conversion of a fatty acid to a nitrile by treating with ammonia, followed by catalytic hydrogenation of the nitrile to primary, secondary, or tertiary amine by suitable adjustment in the reaction conditions. Tallow amines, as well as di(hydrogenated tallow) amine, are commercially available: for example, di(hydrogenated tallow) amines are available under the trade name ARMEEN 2HT^{TM} (Akzo Chemicals, Chicago, Illinois).

Various routes exist for the preparation of phthalamic acids and phthalamic acid salts. In U.S. Patent No. 4,402,708 N,N-diarachidyl phthalamic acid was prepared by adding phthalic anhydride to a 40% solution of amine in toluene in a 1/1 mole ratio at 80°C. The product was recovered by vacuum drying at 50°C, 0.05 mmHg for 20.5 hours. Phthalic anhydride sublimation was observed. This method makes no mention of the presence of any ammonium salt in the resultant product.

U.S. Patent No. 4,402,708 describes a method for preparing N,N-diotadecyl phthalamic acid dioctadecyl ammonium salt and N,N-diarachidyl phthalamic acid diarachidyl ammonium salt. Phthalic anhydride was added to a 10% solution of amine in toluene in an anhydride to amine mole ration of 1/2. The product was recovered by filtering and film evaporating a 1/1 toluene/n-heptane solution at 55°C, 44 mmHg.

Phthalamic acids have also been prepared by melting phthalic anhydride at 131°C and subsequent addition of molten secondary amine. The reactants to be added in an equimolar ratio. At the temperature used in this method, 131°C, excessive phthalic anhydride sublimation occurs and increased product degradation is observed. This method makes no mention of the presence of any ammonium salt in the resultant product.

Phthalamic acids have been prepared by addition of a solution of secondary amine in isopropanol at 78°C to a phthalic anhydride/isopropanol slurry at 78°C in a one to one phthalic anhydride/amine molar ratio with subsequent vacuum stripping of the solvent. This method utilizes isopropanol as the solvent for the reaction. Isopropanol, a secondary amine, reacts with phthalic anhydride to yield isopropyl mono ester of phthalic acid. At 78°C, as much as 40% of the product may be this ester.

Each of these methods produces a mixture of the desired phthalamic acid and an ammonium phthalamate salt. However, these methods make no mention of the presence of any ammonium salt in the resultant product.

However, while various amidocarboxy compounds, including certain fatty phthalamates may be suggested in certain arts, and some fast-breaking emulsions may be suggested for personal-care products as well as some latex polymerization surfactants capable of providing water-insensitive films or coatings may be known, the surfactant arts have not hereto recognised the specific materials, synthesis routes and uses disclosed herein and which exhibit superior surfactant/ emulsification characteristics useful in a wide variety of technical applications including surfactants per se, caustic-stable surfactants, chlorine-stable surfactants, fast-break emulsifiers for personal-care products, emulsifiers for agricultural chemicals, domestic fabric softeners, destructible latex polymerization surfactants, domestic detergent additives, emulsifiers for portland cement and concrete, flotation/benefication additives for various mineral ores, additives for electroplating and/or surface finishing baths for metal goods, additives for plaster, gypsum and miscellaneous building materials, additives for enhanced oil recovery formulations, wetting and lubricating surfactants for textile processing, pulp digestive additives, surfactants for polyurethane/ isocyanurate foam formulation, pour-point depressant for transporting viscous petroleum oils, industrial surfactants for emulsifying a wide variety of oily materials, as suspending agents for various particular materials, etc.

The present invention provides a composition maintained as an emulsion of an oil phase and a water phase, characterised in that the emulsion comprises an emulsifier of the formula: 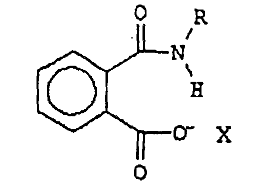
where R represents C₁₆-C₁₈ alkyl;
X represents a sodium or triethanolammonium ion; and the composition is at a pH of 7 to 10.
A preferred composition includes R as C₁₈ alkyl and X as ether, sodium ion or triethanolammonium. Also preferred is the composition wherein X is sodium ion and R is C₁₆ alkyl.

In a preferred embodiment the composition may further comprise a glyceryl mono-fatty acid ester.

The emulsifier may be present in the composition in an amount of at least about 0.5% by weight. The emulsifier may particularly preferably be sodium N-octadecyl phthalamate.

Generally speaking, the weight ratio of the oil phase to the emulsifier may be at least 6.67:1.

Such compositions according to the invention are capable of being fast-breaking emulsions having a breakdown time of less than 30 seconds.

In the composition of the invention the group R represents C₁₆-C₁₈ alkyl, which may be linear or branched, substituted or unsubstituted. The cation X represents a sodium or triethanolammonium ion.

Specific preferred exemplary embodiments of this feature of the invention include:
sodium N-octadecylphthalamate, sodium N-hexadecylphthalamate, sodium N-tallowphthalamate, sodium N-cocophthalamate, sodum N-isododecyloxypropylphthalamate, mixtures thereof and their corresponding triethanolammonium salts. Certain of the surfactants above are caustic-stabile, i.e., function and do not break-down in relatively high pH environments, while certain of the dialkyl surfactants are chlorine-stabile, i.e., function and do not break-down in environments having a relatively high chlorine-ion concentration.

The composition of the invention may be used in the provision of a solvent-tolerant skin-care composition which may comprise about 0.01 percent to about 10.0 percent of a primary emulsifier having the formula above, about 0.01 percent to about 10.0 percent of a secondary emulsifer, and a sufficient amount of an emollient to attain a ratio of emollient to emulsifier in the range of about 3:1 to about 30:1. In certain forms of this embodiment, the skin-care compositions may be relatively salt-tolerant.

The compositions may also be used to provide a hair-care composition comprising about 0.01 percent to about 10.0 percent of a conditioning surfactant having the formula above, about 10 percent to about 25 percent of a hair-cleansing material; and Q.S. 100 of an aqueous material.

Specific exemplary embodiments of conditioning surfactants for hair-care products or compositions include materials selected from the group consisting essentially of sodium N-octadecylphthalamate, sodium N-hexadecylphthalamate, sodium N-tallowphthalamate, sodium N-cocophthalamate, sodium N-isododecyloxypropylphthalamate, mixtures thereof and their corresponding triethanolammonium salts and mixtures thereof.

A particularly attractive use of the invention comprises applying to human skin a relatively salt-tolerant, relatively solvent-tolerant, and relatively fast-breaking anionic oil-in-water cosmetic composition having a pH in the range of 7 to 10, with such composition being comprised of about 0.01 percent to about 10.0 percent of a primary, relatively high HLB emulsifier having the formula above; about 0.01 percent to about 10.0 percent of a secondary, relatively low HLB emulsifier, and a sufficient amount of an emollient to attain a ratio of emollient to emulsifier in the range of about 3:1 to about 30:1 at a select skin area; and rubbing the composition into the skin area for a relatively brief period of time sufficient to break the composition and allow the emollient to penetrate into the skin area. A somewhat similar embodiment of this feature of the invention comprises conditioning or treating hair (to avoid tangles, snarls, etc.) by applying to wet hair a shampoo composition comprised of about 0.05 percent to about 10.0 percent of a water-dispersable anionic surfactant having the formula above; about 10 percent to about 25 percent of a hair-cleansing material; and Q.S. 100 of aqueous material; admixing such shampoo composition with the wet hair and rinsing the resultant hair with water or aqueous solution so as to substantially remove any free shampoo composition.

The invention may also be used to provide an emulsified oil-in-water composition comprising about 0.01 to about 10.0 percent of a relatively high HLB emulsifier having the formula above; about 0.01 percent to about 10.0 percent of a relatively low HLB emulsifier; up to about 80 percent of an oily material and Q.S. 100 water. The oily material may be preferably selected from a group consisting essentially of crude petroleum oil, distilled petroleum oil, heavy paraffinic oil, asphaltene oil, linseed oil, tall oil, soybean oil alkyd, linseed oil alkyd, mineral oil, petrolatum, petrolatum light, isopropyl palmitate, isopropyl myristate, caprylic/capric triglyceride, lanolin, acetylated lanolin alcohol, dimethicone, hydrogenated vegetable oil, sesame oil, safflower oil, avocado oil, corn oil, peanut oil, glycerine, propylene glycol, sorbitol, C₁₂-C₁₆ alcohol benzoates, cyclomethicone, dimethicone, cocoa butter, vitamin E acetate, squalane, sodium pyrolidone carboxylic acid, methyl glucose ether, panthenol, melanin, aloe vera oil, polybutylene, polyethylene, silicone, polysilicone, gum, mixtures thereof, etc.

Another use of the invention is to provide an emulsifier having the formula above, for agricultural chemicals and agricultural chemical compositions comprising, in combination, an amount up to about 10 percent, by weight of the above emulsifier and an effective amount of an active agricultural chemical in a suitable carrier, such as an oil or an aqueous system. A feature of this embodiment of the invention involves applying an active agricultural herbicide/pesticide to a growing crop so as to avoid post-application washoff and comprises producing a sprayable emulsion containing an effective amount of an active agricultural herbicide/pesticide in a suitable carrier and an amount of up to about 10 percent of a destructible surfactant having the general formula above; spraying such eulsions onto the growing crop; and subjecting the sprayed emulsion to ambient temperature conditions over a period of time whereby the surfactant chemically changes so that the resulting agricultural chemical composition becomes substantially water-insensitive. Also, the desired chemical change may be accomplished by subjecting the sprayed crop to a pH environment of less than about 5.

In a further embodiment the invention provides a method of transporting relatively high viscosity crude petroleum oil which comprises forming a relatively low viscosity emulsion from a select crude petroleum oil, and an emulsifier having the formula above, and water, and transporting, as by a pump or truck, such emulsion to a desired location, and, at such a location, subjecting such emulsion to conditions sufficient to chemically change the emulsifier to "deactivate" such emulsifier and thereby break the emulsion and allow ready separation of the oil from the admixture. In certain embodiments of the foregoing feature of the invention, conditions sufficient to chemically change the emulsifier so as to deactivate the same involve heating the emulsion to temperatures of at least about 100°C. In yet certain other embodiments of this feature of the invention, conditions sufficient to chemically change the emulsifier so as to deactivate the same, involve adjusting the pH of the emulsion to a value below about 5. A further preferred embodiment of this feature of the invention includes recovering the chemically changed (deactivated) emulsifier and adjusting the pH of the so-recovered deactivated emulsifier to a value above about 7.5 so as to "reactivate" said emulsifier for further use in transporting crude petroleum oil.

A further use of the invention is to provide an emulsifier/dispersant for crude petroleum oil, with the emulsifier/dispersant having the composition above.

Other and further features, objects and embodiments of the invention will now be apparent to those skilled in the art from the following description and claims.

The surfactant solutions of the present invention may be used as surfactants per se, as caustic-stabile surfactants, as chlorine-stabile surfactants, as cosmetic emulsifiers for personal care products, such as skin creams, skin lotions, hair conditioners, etc., as fast-breaking skin-care product emulsifiers; as emulsifiers for liquid hand, facial and body soaps and bar soaps; as emulsifiers for agricultural chemicals, as domestic fabric softeners; as destructible latex polymerisation surfactants in coating and/or adhesive systems, as domestic detergent additives, such as in heavy-duty detergents, in light-duty detergents, in dishwashing detergents, in various hard-surface cleaners, etc., as emulsifiers for portland cement and concrete; as flotation/benefication additives for various mineral ores; as additives for electroplating and/or surface finishing baths for metal goods; as additives for plaster, gypsum and miscellaneous building materials; as enhanced oil recovery additives; as wetting, lubricating, and penetrating surfactants for textile processing; as pulp digestive additives; as surfactants for polyurethane/isocyanurate foam systems; as pour-point depressants for transporting viscous petroleum oils; as industrial surfactants for emulsifying a wide variety of oily materials or oliginous materials such as linseed oils, alkyd resins, polybutylenes, silicones, polysilicones, silicone gums, etc.; as low temperature stabilizers for fatty alcohol/ water emulsions; as suspending agents for various particulate materials, such as coal tar, sulfur or coal; etc.

In certain embodiments of the invention, the R moieties in the formula above, are typically hydrocarbon moieties and may include moieties wherein one or more of the carbon atoms in the respective carbon chains are replaced by a hetero atom, such as, for example, oxygen and/or nitrogen. Further, in certain embodiments of the invention the aromatic moiety in the above formulation may be substituted with various functional groups, which include but are not limited to, halides, carboxylic acids and derivatives thereof, nitro, nitroso, amines, amidos, hydroxyls, sulfonic acids and derivatives thereof, alkyls, ethers, esters, nitriles and mixtures thereof.

Preferred compositions have many technical utilities, including as cleansers, emulsifiers, wetting agents, lubricants, surfactants, etc., in such diverse applications as caustic-stabile surfactants useful, for example, in textile scouring baths or textile spinfinishing operations; chlorine-stabile surfactants useful, for example, in hard-surface cleaners containing bleach, or in liquid automatic dishwashing detergents, or in domestic laundry cleansing formulations containing chlorine, etc.; as foodstuff emulsifiers useful in emulsifying various edible oils and the like into foodstuff compositions, such as baked goods, mayonnaise, dog/cat food, etc.; as emulsifiers for skin/hair-care products and useful in emulsifying various emollients or conditioning/protective agents used in treating skin and/or hair; as fabric softeners for treating domestic laundry; as industrial emulsifiers useful in emulsifying various oily materials, such as crude petroleum oils, linseed oils, vitamin acetates, etc., including agricultural chemicals such as oil or water soluble or dispersable pesticides and/or herbicides; as latex polymerisation surfactants useful in producing water-insensitive pigment and/or adhesive coatings, etc.; as pulp digestive additives; as polyurethane/ polyisocyanurate foam surfactant additives, etc. Exemplary specifically preferred embodiments of the invention include:

sodium N-octadecylphthalamate, sodum N-hexadecylphthalamate, sodium N-tallowphthalamate, sodium N-cocophthalamate, sodum N-isododecyloxypropylphthalamate.

Embodiments of the invention include synthesis routes useful in manufacturing surfactants of the formula above at relatively high yields, with little or no purification requirements.

In certain of these embodiments, molten phthalic anhydride may be contacted with at least a primary (RNH₂) amine, wherein R is selected from C₁₆-C₁₈ alkyl, under reaction conditions conducive for formation of an imide between the anhydride and the amine, and thereafter adding to the resulting imide, a nucleophilic source yielding an alkali (i.e. Na, triethanolammonium) cation under reaction conditions conducive to the formation of the corresponding alkali salt. Reaction conditions conducive to formation of the imide and the desired salt generally include applying heat in the range of about 90° to about 150°C while stirring, preferably under an inert atmosphere, such as nitrogen, over a period of time, typically at least about 30 minutes and less than about 300 minutes. During imide formation, water may be removed and during salt formation water may be added.

In certain other of these embodiments, surfactants of the formula above may be produced by contacting molten phthalic anhydride with at least a secondary R₅R₆NH) amine where R₅ and R₆ are independently selected from the group consisting essentially of H or C₁₆-₁₈ linear or branched, substituted or unsubstituted, alkyl, cycloalkyl, alkylene, alkaryl, aryl, or R₃-O-R₄ groups, wherein R₃ and R₄ are defined as for R₅ and R₆, under reaction conditions conducive for formation of phthalamic acid between the anhydride and the amine, and thereafter adding a source yielding a cation selected from the group consisting essentially of Na or (HOCH₂CH₂)₃NH, and mixtures thereof under reaction conditions conducive for formation of the corresponding salt.

In yet certain other of these embodiments, surfactants of the formula above, may be produced by contacting phthalic anhydride with at least a tertiary C₁₆₋₁₈ amine so as to obtain an admixture, typically a substantially uniform admixture, and adding to such admixture a primary or secondary (R₅R₆NH) amine, with R₅ and R₆ being independently selected from the group consisting essentially of H or C₁₆_₁₈ linear or branched, substituted or unsubstituted, alkyl, cycloalkyl, alkylene, alkaryl, aryl, or R₃-O-R₄ groups, wherein R₃ and R₄ are defined as above, under reaction conditions conducive for formation of an alkylphthalamate salt.

In certain reaction schemes, the foregoing steps may occur sequentially or substantially simultaneously.

With the foregoing general discussion in mind, there will now be presented a number of detailed exemplary embodiments of the invention and workers skilled in the art will appreciate that the following examples are non-limitive embodiments of the invention and are included merely as specific exemplification of the invention.

### EXAMPLE I

### Synthesis of Alkylphthalamate With Primary Amines

A number of alkylphthalamate surfactants are produced from phthalic anhydride and various fatty primary amines in accordance with the following reaction scheme: 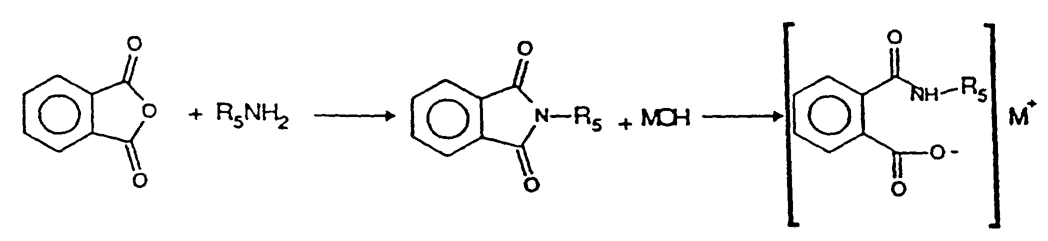 wherein R_{*5*} is selected from C_{*1*}-C*₄₀* linear or branched, substituted or unsubstituted alkyl, cycloalkyl, alkytene, alkaryl, aryl, or R_{*3*}-O-R_{*4*} groups, with R_{*3*} and R_{*4*} being independently selected from C_{*1*} - C_{*22*} linear or branched, substituted or unsubstituted alkyl, cydoalkyl, alkylene, alkaryl, and aryl groups and M is an alkali cation (i.e. Na, K,,NH_{*4*}, etc.).

In all synthesis runs of this Example, select reactants may be placed in a suitable reaction flask, which typically may be fitted with a stirrer, and Allihn condenser, a Dean-Stark trap, a controlled inlet interconnected to a nitrogen gas source, a thermometer and an addition funnel, or their equivalent. Phthalic anhydride may be placed in the reactor flask, heated to a melt (about 131°C), generally under a nitrogen blanket, and an amount of a select fatty amine may be brought into contact with the molten phthalic anhydride. Heating may be continued over a period of time ranging from at least about 30 minutes to about 300 minutes, with removal of water aided by a nitrogen or other inert gas sparge. Thereafter, deionized water may be added and the temperature of the reaction mixture may be adjusted to about 100°C and a select nucleophilic source, i.e., a hydroxide, yielding a desired cation may be added relatively quickly. This reaction mixture may be heated with stirring for a period of time varying from at least about 30 minutes to about 300 minutes to obtain a high yield, typically about 90% or more, of an alkylphthalamate without requiring purification. Of course, if desired, purification may be effected.

Phthalic anhydride is a well-known chemical and is commercially available, for example from Stepan Company, Northfield, Illinois, (assignee of the present invention). Fatty primary amines are likewise well known and are commercially available from various sources, including, for example, Aldrich Chemical Company.

Exemplary fatty primary amines include: alkyl, cydoalkyl, and/or alkylene amines, such as hexylamine, cyclohexolamine octylamine, decylamine, dodecylamine, tetradecylamine, hexadecylamine octadecylamine, eicosylamine, docosylamine, cocoamine, tallowamine, and similar linear or branched, substituted or unsubstituted alkyl, cycloalkyl or alkylene fatty amines; alkaryl amines, such as 4-methylaniline, 2,4-dimethylaniline, 3,4-diisopropylaniline, 3,5-dimethylaniline, and similar linear or branched, substituted or unsubstituted alkaryl fatty amines; aryl amines, such as aniline, 2-bromoaniline, 2-chloroaniline, 3-bromoaniline, 4-chloroaniline, 3,4-diethylaniline, 3,4,5-tribromoaniline and similar substituted or unsubstituted aryl fatty amines; fatty ether amines, such as isododecyloxypropylamine, isodecyloxypropylamine, hexyloxypropylamine, and similar fatty ether amine compounds.

Exemplary nucleophilic sources yielding alkali cations include: NaOH, KOH, NH_{*4*}OH. Exemplary sources yielding alkaline-earth metal cation indude: Ca(OH)_{*2*}, Ba(OH)_{*2*}, Mg(OH)_{*2*}. Exemplary sources yielding Al, Ti, or Zr cations indude: Al[OCH(CH_{*3*})_{*2*}]_{*3*}, Ti(OCH_{*2*}CH_{*2*}CH_{*3*})_{*4*}, Zr(OCH_{*2*}CH_{*2*}CH₃)_{*4*} and similar alkoxide compounds.

Specific synthesis of sodium N-octadecylphthalamate, ammonium N-dodecylphthalamate, potassium N-cocophthalamate, sodium N-dodecylphthalamate, sodium N-isododecyloxypropylphthalamate, calcium N-octadecylphthalamate and aluminum N-octadecylphthalamate will now be set forth and workers in the art will recognize that similar-syntheses may be performed to attain a desired alkyl, cycloalkyl, alkylene, dialkyl, dialkylene, alkaryl, aryl, etc. phthalamate salt

### EXAMPLE 1A

### Sodium N-Octadecylphthalamate

Phthalic anhydride (1 mole, 148g) was placed in a two liter, three neck, round bottom reaction flask fitted with a magnetic stirrer, an Allihn condenser, a Dean-Stark trap, an inlet for nitrogen, a thermometer and an addition funnel. The phthalic anhydride was heated under a nitrogen blanket to a melt (about 131°C). Octadecylamine (1 mole, 278g) was added slowly over about 30 minutes to the molten phthalic anhydride. Heating was continued at about 131°C for about 3 hours, with the nitrogen blanket changed to a nitrogen sparge to aid in the removal of water. A clear pale yellow liquid was obtained, from which a small sample was withdrawn, allowed to solidify (off-white) and characterized by IR spectroscopy (Beckman 983 spectrometer) as comprising N-octadecylphthalimide.

One mole (408g) of this reaction product, N-octadecylphthalimide, was transferred into a twelve liter, 4-neck, round bottom flask fitted with a magnetic stirrer, an Allihn condenser, an addition funnel and an inlet for connection to a nitrogen source (gas cylinder). Deionized water (about 6.1L) was added to the N-octadecylphthalimide and the mixture was heated to about 100°C to obtain a fairly homogenous mixture. Stirring was then commenced under a nitrogen blanket and 1 N sodium hydroxide (about 1.1L 10% excess) was added relatively quickly over a period of a few minutes. The reaction mixture was stirred with heating for about three hours. A clear light yellow reaction product was obtained and a relatively small sample was removed, dried in a vacuum oven for about 1 hour (1 atm, 40°C) and characterized by IR spectroscopy as comprising essentially sodium N-octadecylphthalamate.

### EXAMPLE 1B

### Ammonium N-Dodecylphthalamate

The above synthesis is, essentially, duplicated except that dodecylamine is substituted for octadecylamine and ammonium hydroxide is substituted for sodium hydroxide. The final product is characterized by IR spectroscopy as comprised essentially of ammonium N-dodecylphthalamate.

### EXAMPLE 1C

### Potassium N-Cocophthalamate

The synthesis route of Example 1A above is repeated, except that cocoamine is utilized in place of octadecylamine and potassium hydroxide is used in place of sodium hydroxide. The final product is similarly characterized as comprising essentially potassium N-cocophthalamate.

### EXAMPLE 1D

### Sodium N-Dodecylphthalamate

The synthesis route of Example 1A was essentially repeated, except that dodecylamine was utilized in place of octadecylamine. The final product, straw yellow, was characterized by IR spectroscopy as comprising sodium N-dodecylphthalamate. A sample of the reaction product was removed and weighed 52.2980 g as about 11.3 percent solids in water. This sample was quantitively converted to the corresponding phthalamic acid and using conventional calculations, it was determined that the percent yield of the acid (or percent purity of the sample) was about 94.16 percent.

### EXAMPLE 1E

### Sodium N-Isododecyloxypropylphthalamate

The synthesis route of Example 1A was, essentially, repeated except that isododecyloxypropylamine was used in place of octadecylamine and hydrolysis with NaOH occurred at about 70°C, instead of 100°C. The final product was characterized by IR spectroscopy as comprising essentially of sodium N-isododecyloxypropylphthalamate.

### EXAMPLE 1F

### Calcium N-Octadecylphthalamate

A portion of the sodium N-octadecylphthalamate from Example 1A above is acidified with a mineral acid, i.e., hydrochloric acid, to a pH of about 3 and filtered to separate the semi-solid acid. This acid is then admixed with deionized water and a slurry of calcium hydroxide is added, with stirring until a relatively stabile pH of about 8 or 9 is attained. The final product is confirmed by IR spectroscopy as comprising essentially of calcium N-octadecylphthalamate.

Similarly, the calcium salt may also be obtained by adding calcium hydroxide directly to the alkali alkylphthalamate. Also a magnesium or barium salt may be produced by substituting Mg(OH)_{*2*} or Ba(OH)_{*2*} for the Ca(OH)_{*2*} in the above neutralization scheme.

Neutralization of alkyl and/or alkylene phthalamic acids may be accomplished in water or in a non-aqueous solvent, such as methanol, isopropanol, or the like, by addition of a suitable base (including various amines) to obtain a pH of about 8 or 9. When a neat final product is desired, a low-boiling, non-aqueous solvent such as methanol, isopropanol or the like may be used, with subsequent easy removal of the solvent by vacuum stripping.

### EXAMPLE 1G

### Aluminum N-Octadecylphthalamate

Aluminum isopropoxide (0.0117 moles, 2.38g) and about 200 mL of isopropanol were placed in a 1L four-neck, round bottom reaction flask provided with a nitrogen sparge, an Allihn condenser, a magnetic stirrer and a thermometer. This mixture was heated to a temperature of about 78°C, with stirring for about 1 hour. Then N-octadecylphthalamic acid (0.0353 moles, 15.0g)-obtained via the procedure of Example 1A and acidified with HCl to a pH of about 3 and filtered for separation - was slowly added as a powder. The resultant mixture was heated at reflux (about 78°) for about 3 hours. Upon confirmation of the reaction product by IR spectroscopy, the isopropanol was stripped-off under vacuum. A white solid final product was obtained.

### EXAMPLE II

### Synthesis of Alkylphthalamates With Secondary Amines

A number of alklyphthalamates are produced from phthalic anhydride and various fatty secondary amines in accordance with the following reaction scheme: 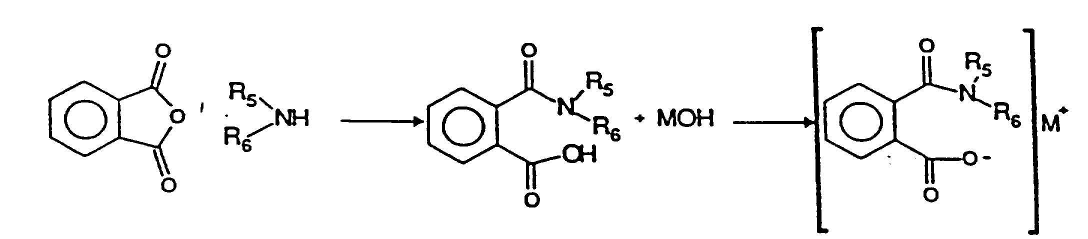 wherein R_{*5*} and R_{*6*} are independently selected from C_{*1*}-C_{*40*} linear or branched, substituted or unsubstituted alkyl, cycloalkyl, alkylene, alkaryl, aryl, or R_{*3*}-O-R_{*4*}, groups with R_{*3*} and R, being independently selected from C_{*1*} - C_{*22*} linear or branched, substituted or unsubstituted alkyl, cycloalkyl, alkylene, alkaryl, and aryl groups and M is a cation selected from Na, K, NH_{*4*} [including (CH_{*3*}CH_{*2*})_{*3*}NH, (CH_{*3*}CH_{*2*})_{*2*}NH_{*2*,} (HOCH_{*2*}CH_{*2*})_{*3*}NH, (HOCH_{*2*}CH_{*2*})_{*2*}NH_{*2*} and similar ammonium derivatives], Ba, Ca, Mg, Al, Ti, Zr, and mixtures thereof.

The equipment and conditions for the synthesis runs of this Example are, essentially, identical to those described in Example I above. (Except that the Dean-Stark trap may be eliminated).

Phthalic anhydride is a well known chemical compound and is commercially available, for example, from Stepan Company, Northfield, Illinois (the assignee of the instant invention). Fatty secondary amines are likewise known and are commercially available from various sources, for example, Aldrich Chemical Company.

Exemplary fatty secondary amines indude: alkyl, cycloalkyl, and/or alkylene amines, such as; N-dodecyl-N-methylamine, N-tetradecyl-N-methylamine, N-hexadecyl-N-methylamine, N-octadecyl-N-methylamine, N-methyl-N-cyclohexalamine, N-coco-N-methylamine, N-tallow-N-methylamine, N,N-dicocoamine and similar linear or branched, substituted or unsubstituted alkyl, cycloalkyl, or alkylene fatty secondary amines; alkaryl amines, such as; N-methyl-4-dodecylaniline, N-methyl-4-octadecylaniline, N-methyl-4-hexadecylaniline, N-methyl-4-tallowaniline, N-methyl-4-cocoaniline and similar linear or branched, substituted or unsubstituted alkaryl secondary amines; aryl secondary amines, such as: N-methylaniline, N-propylaniline, and similar substituted or unsubstituted and aryl secondary amines.

Exemplary sources yielding select M cations include: NaOH, KOH, NH_{*4*}OH, Ca(OH)_{*2*}, Ba(OH)_{*2*}, Mg(OH)_{*2*}, Al[OCH(CH_{*3*})_{*2*}]_{*3*}, Ti(OCH_{*2*}CH_{*2*}CH_{*3*})_{*4*}, Zr(OCH_{*2*}CH_{*2*}CH_{*3*})_{*4*} and similar sources.

Specific syntheses of sodium N-dodecyl-N-methylphthalamate and triethanolammonium N,N-dicocophthalamate will now be set forth and workers skilled in the art will recognize that similar synthesis routes may be performed to obtain other desired phthalamate salts.

### EXAMPLE IIA

### Sodium N-Dodecyl-N-methyphthalamate

Phthalic anhydride (0.10 mole, 14.8g) was placed in a 500 mL reaction flask similarly configured and equipped to that described in Example 1A. The phthalic anhydride was heated to a melt (131°C) and N-dodecyl-N-methylamine (0.10 mole, 19.9g) was added to the molten anhydride over a period of about 15 minutes. Heating was continued at about 131°C for about 3 hours. A clear amber, viscous liquid was obtained, which was characterized by IR spectroscopy as N-dodecyl-N-methylphthalamic acid. This reaction product (about 30g) was diluted with methanol (ca. 50g) and neutralized with a methanolic sodium hydroxide (1 N) to a pH of about 8.65. The methanol was then stripped off using a rotary evaporator (water aspirator, 30°C bath). A white powder was obtained and was characterized by IR spectroscopy as essentially comprising of sodium N-methyl-N-dodecylphthalamate. Methanol was utilized for sake of convenience in producing a 100 percent active material. Water may be utilized if desired to obtain lower active material or if higher temperature, vacuum, etc., are utilized.

### EXAMPLE IIB

### Triethanolammonium N,N-Dicocophthalamate

Phthalic anhydride (0.2 moles, 29.6g) was placed in a reaction flask configured and equipped similarly to that described above in Example IIA, heated to a melt and N, N-dicocoamine (0.2 moles, 76.6 g) was added over a relatively brief time period. Heating was continued at about 131°C for about 3 hours. A liquid reaction product was obtained and characterized by IR spectroscopy as comprising essentially N,N-dicocophthalamic acid. About 30g of this acid was diluted with water (ca. 50g) and neutralized with triethanolamine to a pH of about 9. The water was then stripped-off as before and the white paste obtained was characterized by IR spectroscopy as comprising essentially triethanolammonium N,N-dicocophthalamate.

When a non-aqueous product is desired, neutralization can occur in a non-aqueous solvent, such as methanol or isopropanol, which can then be removed after neutralization by vacuum.

### EXAMPLE III

### Synthesis of Alkylphthalamate with Tertiary Amines

A number of alkylphthalamate surfactants may be produced from phthalic anhydride and various fatty primary or secondary amines in the presence of a tertiary amine in accordance with the following reaction scheme: 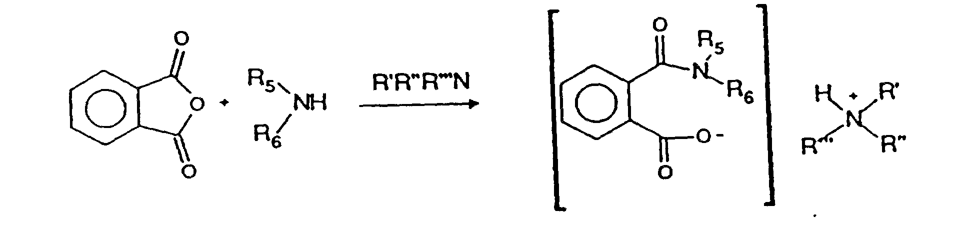 wherein R_{*5*} and R_{*6*} are independently selected from H or C_{*1*} - C_{*40*} linear or branched, substituted or unsubstituted alkyl, cycloalkyl, alkylene, alkaryl, cydic including aryl, or R_{*3*}-O-R_{*4*} groups, with R_{*3*} and R₄ being independently selected from C_{*1*} - C_{*22*} linear or branched, substituted or unsubstituted alkyl, cycloalkyl, alkylene, alkaryl and aryl groups and R', R", R‴ are independently selected from C_{*1*} - C_{*40*} linear or branched, substituted or unsubstituted alkyl, cycloalkyl, alkylene, alkaryl, aryl or R_{*3*}-O-R_{*4*} groups, with R_{*3*} and R_{*4*} being as defined above.

When a cation other than an ammonium derivative is desired, the above-obtained reaction product may be acidified as set forth earlier, isolated, and then neutralized with a suitable source yielding the desired cation, i.e. Na, K, Ca, Ba, Mg, Al, Ti, Zr or mixtures thereof.

The equipment for the synthesis runs of this Example is, essentially, identical to that described earlier, except that an ice bath may be required to control the exothermic nature of this reaction.

As indicated earlier, phthalic anhydride is commercially available from Stepan Company, Northfield, Illinois. Primary and/or secondary amines are likewise known and commercially available from various sources as set forth earlier. Tertiary amines are likewise commercially available.

Exemplary primary and secondary amines are set forth above and exemplary tertiary amines include: triethylamine, triethanolamine, trimethylamine, trimethanolamine, N,N-dimethyl cyclohexylamine, N,N-dimethylaniline and similar alkyl, cycloalkyl, alkylene alkaryl and aryl tertiary amines.

Specific synthesis of triethylammonium N-cocophthalamate, N,N-dimethylcyclohexylammonium N-isododecyloxypropylphthalamate and triethalonammonium N-isododecyloxypropylphthalamate will now be set forth and workers skilled in the art will recognize that similar synthesis routes may be followed to obtain other desired phthalamate salts.

### EXAMPLE IIIA

### Triethylammonium N-Cocophthalamate

Phthalic anhydride (0.2 moles, 29.6g) and triethylamine (0.2 moles, 20.2g) were introduced into a 500 mL, three-neck, round bottom flask, equipped with a magnetic stirrer, Allihn condenser, an inlet for nitrogen, a thermometer and an addition funnel. This mixture was stirred until a fairly uniform mixture was attained. Cocoamine (0.2 moles, 40.8 g) was then slowly added at a rate which maintained the temperature of the reaction mixture below about 35°C. After completion of the cocoamine addition, the mixture was stirred for about 1 hour while the temperature of the reaction mixture was maintained at about 30°C. A clear yellow liquid product was obtained, cooled to a pale yellow paste and was characterized by IR as essentially comprising triethylammonium N-cocophthalamate.

### EXAMPLE IIIA - 1

### Triethylammonium N-Cocophthalamate

The procedure of Example IIIA above is repeated except that triethylamine and cocoamine are added substantially simultaneously to the phthalic anhydride. The reaction may be quite exothermic and an ice bath may be utilized to maintain control.

### EXAMPLE IIIB

### N,N-Dimethylcyclohexylammonium N-Isododecyloxypropylphthalamate

The procedure of Example IIIA was, essentially, repeated, except that N,N-dimethylcyclohexylamine was substituted for triethylamine and isododecyloxypropylamine was substituted for the cocoamine. The final product was characterized by IR spectroscopy as comprising essentially of N,N-dimethylcyclohexylammonium N-isododecyloxypropylphthalamate.

### EXAMPLE IIIC

### Triethanolammonium N-Isododecyloxypropylphthalamate

The synthesis route of Example IIIA was, essentially, repeated, except that triethanolammonium was substituted for triethylammonium and isododecyloxypropylamine was substituted for the cocoamine. The final product was characterized by IR spectroscopy as comprised essentially of triethanolammonium N-isododecyloxypropylphthalamate.

### EXAMPLE A

### Emulsion Stability Characteristics

In order to obtain reproducible results, an emulsion stability test, modified from the standard used in the art for testing surfactants was developed. In the modified procedure, 0.5g of a select surfactant (instead of 1g) was dissolved in 90 mL of deionized water in a graduated cylinder containing 5.0 mL of xylene, which was then sealed and rocked between a vertical/horizontal position for 30 cycles, allowed to settle for about 5 to 10 minutes and subjected to the same rocking cycle with the results of the second sequence recorded (instead of the first sequence). The results obtained for various alkylphthalamates are set forth in Table II below:

**TABLE II**

| Compound | Emulsion Break Rate (mL/sec) |
|---|---|
| Sodium N-octylphthalamate | 0.111 |
| Sodium N-decylphthalamate | 0.034 |
| Sodium N-dodecylphthalamate | 0.068 |
| Sodium N-tetradecylphthalamate | 0.085 |
| Sodium N-hexadecylphthalamate | 0.102 |
| Triethanolammonium N-octyphthalamate | 0.043 |
| Triethanolammonium N-decylphthalamate | 0.046 |
| Triethanolammonium N-dodeylphthalamate | 0.039 |

As is apparent from the above data, the triethanolammonium salts exhibited better (i.e., slower) emulsion break rates relative to the sodium salts. However, in personal-care formulations, (which are materially different systems from a xylene/water system) the sodium salts appeared to perform as well or better than the triethanolammonium salts.

| | |
|---|---|
| 1 NEOBEE^{*R*} M-5 = | Stepan Company's registered trademark for caprylic/capric triglyceride. |
| 2 NEOBEE^{*R*} M-20 = | Stepan Company's registered trademark for propylene glycol dicaprylate/dicaprate. |
| 3 NEOBEE^{*R*} 18 = | Stepan Company's registered trademark for safflower oil. |
| 4 WECOBEE^{*R*} = | Stepan Company's registered trademark for hydrogenated vegetable oil. |
| 5 ALPHA-STEP^{*R*} ML-40 | Stepan Company's registered trademark for sodium alphasulfo methyl ethyl laurate. |
| 6 STEPANOL^{*R*} WA-EX = | Stepan Company's registered trademark for sodium lauryl sulfate |
| 7 STEOL^{*R*} CS-330 = | Stepan Company's registered trademark for sodium laureth sulfate. |
| 8 NINOL^{*R*} 40-C = | Stepan Company's registered trademark for N,N-diethanolcocomide. |
| 9 AMMONYX^{*R*}LO = | Stepan Company's registered trademark for lauralamine oxide. |
| 10 AMPHOSOL^{*R*} CG = | Stepan Company's registered trademark for cocamide propyl betaine. |
| 11 MAKON^{*R*} = | Stepan Company's registered trademark for nonylphenol polyethoxylate. |
| 12 STEPANATE^{*R*} X = | Stepan Company's registered trademark for sodium xylene sulfonate. |

### EXAMPLE B

### Anionic Emulsification Characteristics for Personal-Care Formulations

Four typical personal-care formulations were parepared with different N-alkylphthalamates. In each instance, the ingredients listed under Part A were combined to produce a substantially uniform mixture with application of heat in the range of about 74°C to 77°C (165°F to 170°F). Similarly, the ingredients listed under Part B were combined with heat so as to obtain a uniform mixture. Thereafter, the Part A mixture was added to the Part B mixture, with good agitation and continued heating to acheive a substantially uniform mixture. The pH of the final mixture was then adjusted with citric acid (although other acids may be utilized) to a pH in the range of aoubt 7.0 to 8.5. The resultant mixture was then allowed to cool to about 35°C (45°F) and evaluated. As can be seen from the data presented in Table V below, these surfactants exhibit excellent emulsion quality and provide excellent performance quality.

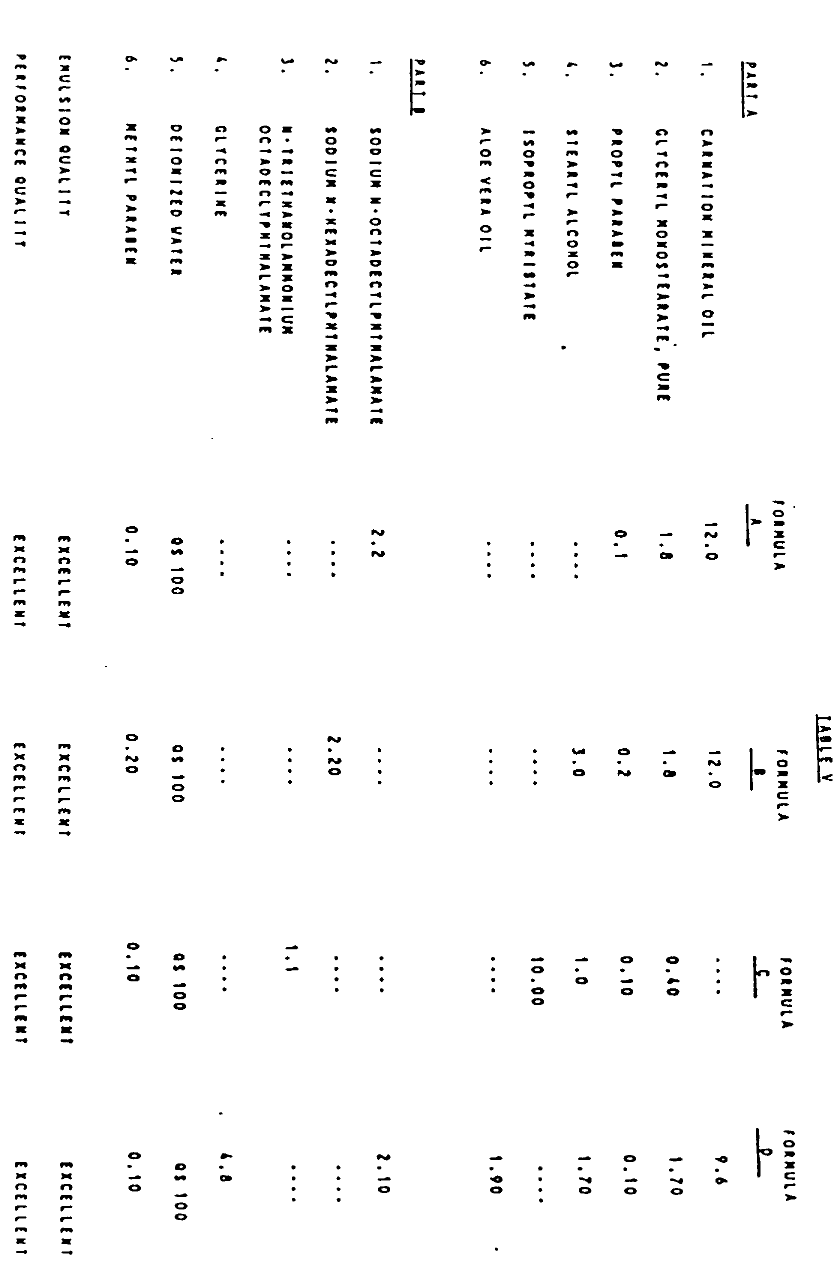

### EXAMPLE C

### Rapid Breaking Emulsification Characteristics for Personal-Care Formulations

A number of N-alkylphthalamates were formulated into typical personal-care compositions and evaluated.

The effective usage level of the N-alkylphthalamates in these compositions is about 0.01% to about 10% by weight, with the preferred level of about 1.0% to about 4.0% An effective pH range for these compositions is from about 6 to about 10, with a preferred pH range being between about 7.6 to about 9.5. While C*₁* - C*₄₀* chain lengths are useful, a preferred chain length is about C*₈* to C*₁₈* and a more preferred chain length was found to be C*₁₆*, tallow (C*₁₆* - C*₁₈*) and C*₁₈*. The respective results are set forth in Tables VI-A through VI-C below.

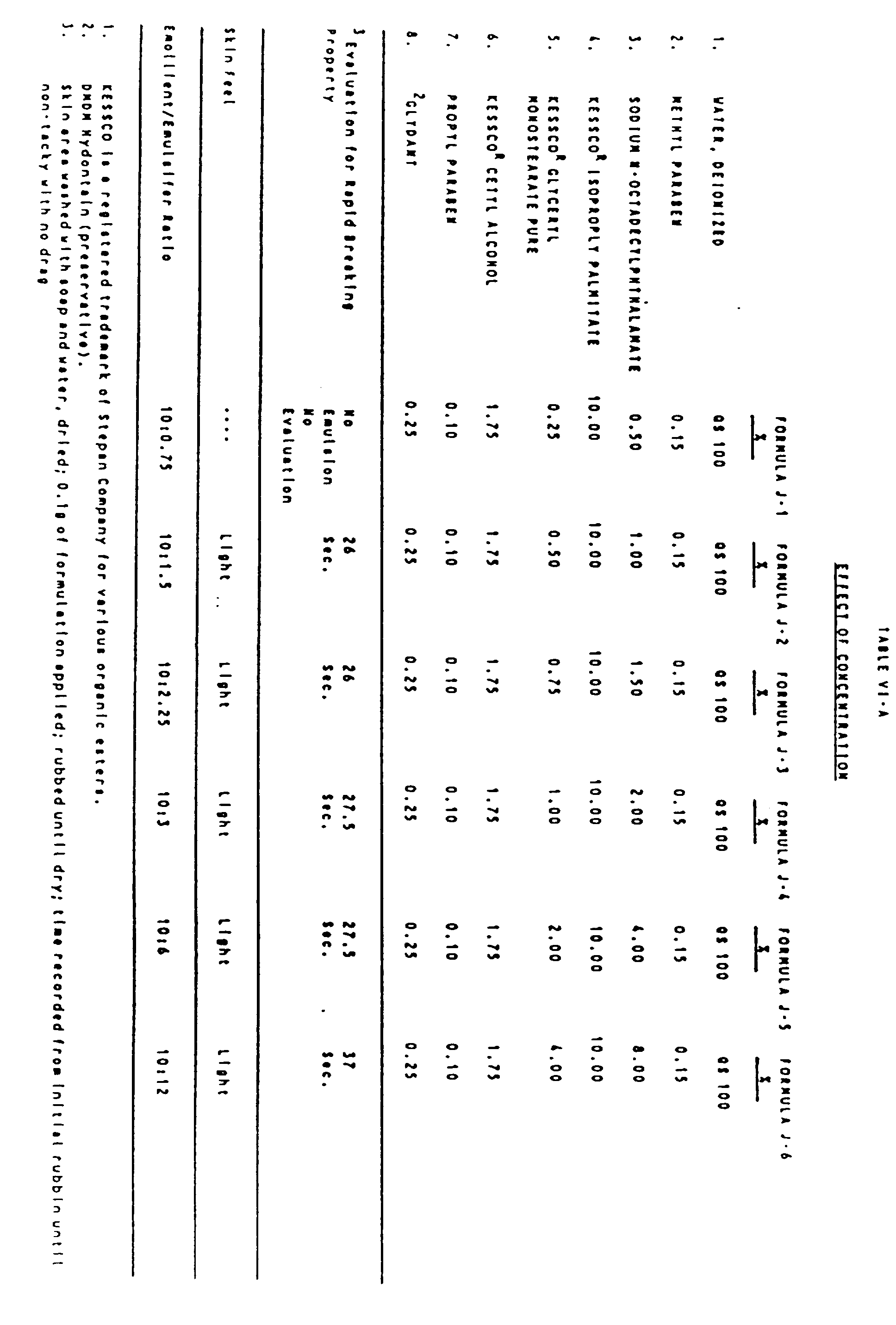

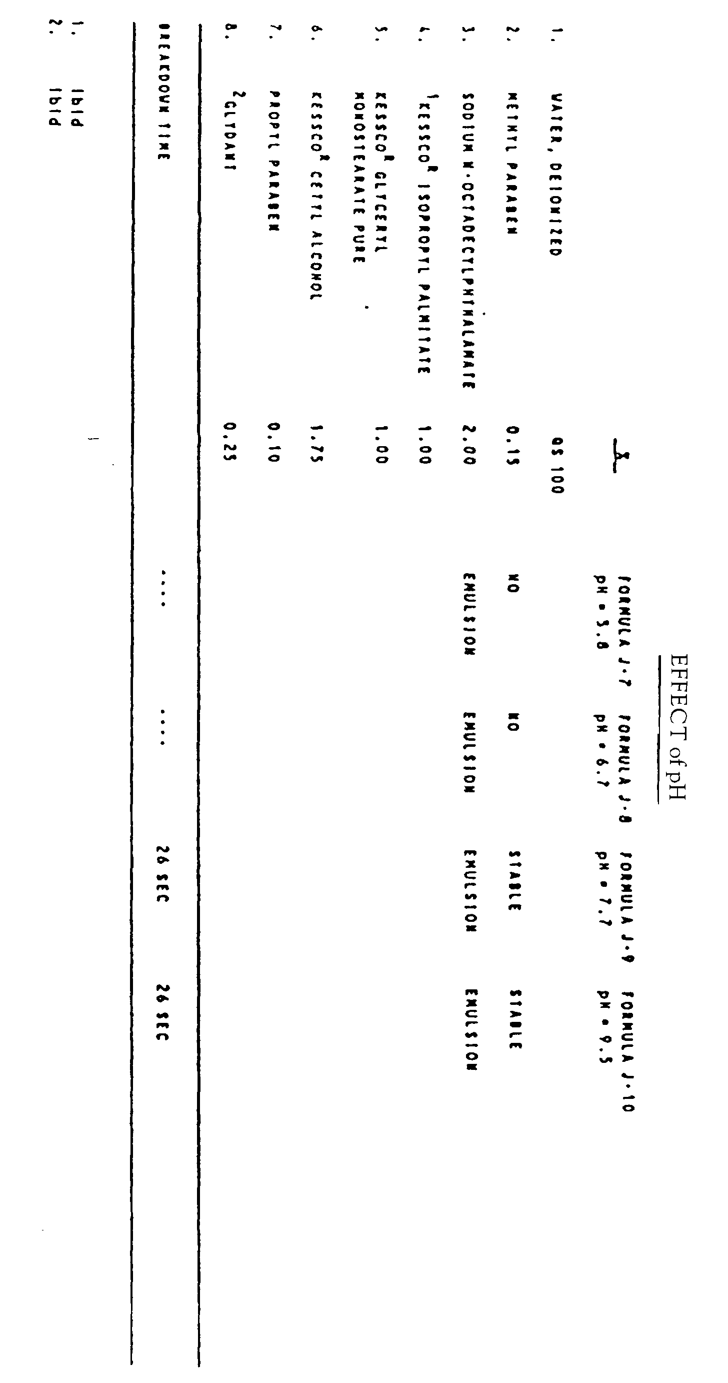

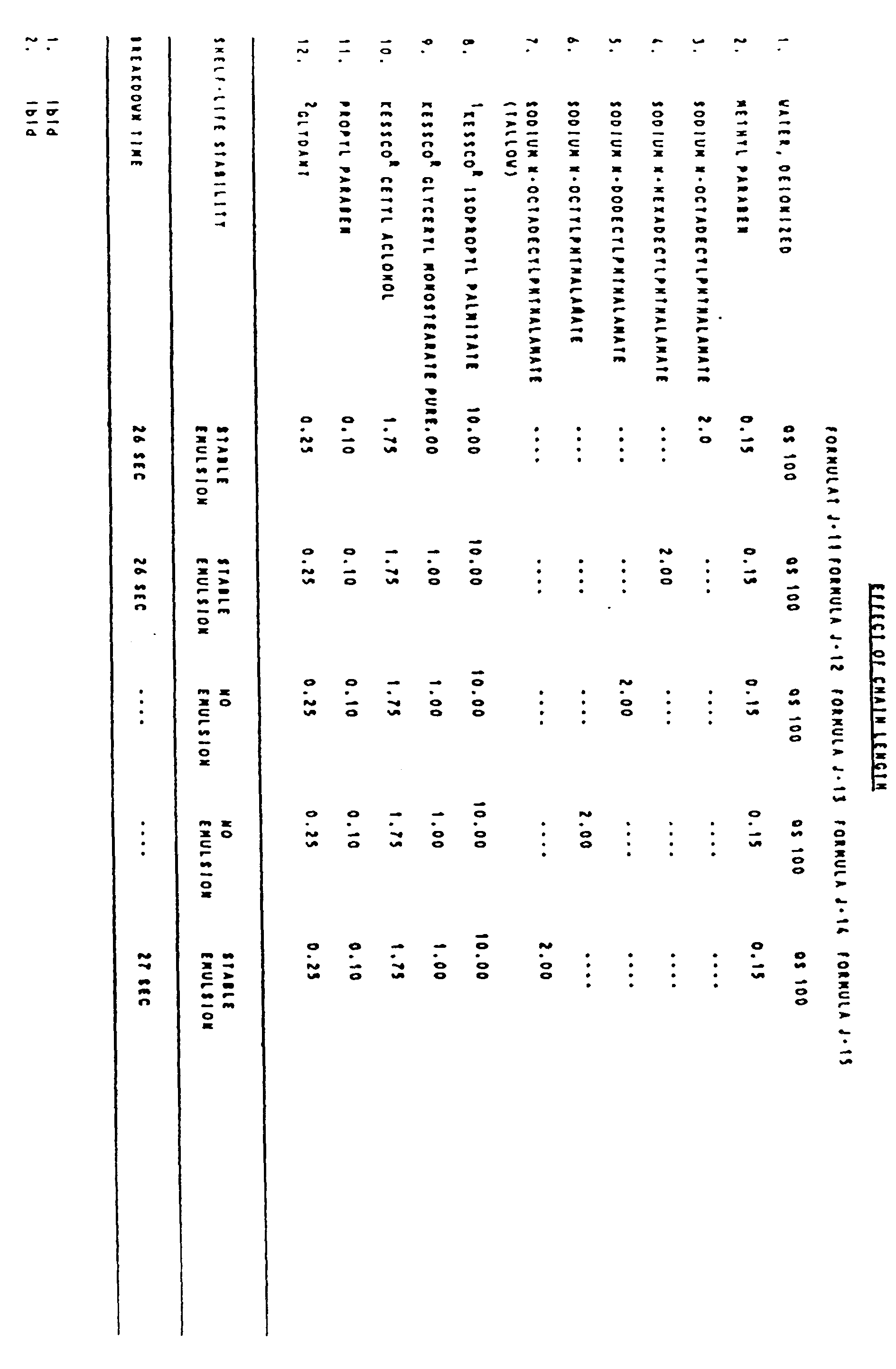

Various commercial personal-care products (skin-care lotions) suggest that their formulations provide quick penetration into skin areas brought into contact therewith. It is known in the emulsion technology art that the faster breaking emulsions are oil-in-water, nonionic or anionic soap-based emulsions which contain Carbopol^{*R*} (a registered trademark of B.F. Goodrich Company for certain polymerized vinyl resins, see EPA 026816A2) as the primary emulsifier. Carbopol^{*R*} materials appear unstabile in the presence of salts (electrolytes) so that when emulsion products containing such materials are applied to skin, which includes salt from perspiration or the like, the emulsion breaks and emollients and the like, in the product then quickly penetrate the skin. Emulsions without Carbopol^{*R*} , regardless of type (anionic or nonionic), do not break down as readily when applied to skin.

The N-alkylphthalamates of the invention comprise excellent cosmetic emulsifiers and are water dispersable (i.e., high HLB) emulsifiers. Hydrophiliclipophilic balance or HLB is a widely accepted measure of the polarity of a surfactant and of its relative affinity for aqueous or hydrocarbon media. It is generally held [see, for example, Nonionic Surfactants, edited by Schick (Marcel Dekker Inc, N.Y. 1967) pages 606-608] that HLB ranges for different surface active functions are as follows:

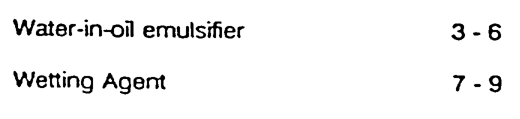

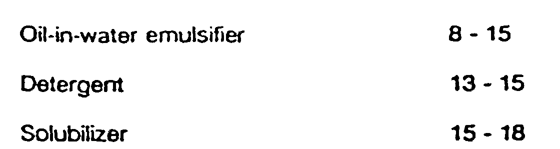

The N-alkylphthalamates of the invention are characterized as being relatively high HLB emulsifiers or primary emulsifiers having an HLB value in the range of about 7 to 15, and when utilized to formulate cosmetic emulsions are combined with secondary or relatively low HLB emulsifiers, such as glyceryl stearate to produce rapidly breaking, loosely emulsified emulsions. The resulting optimal emulsions were found to exhibit excellent shelf-life stability while breaking down extremely quickly when applied to skin. As shown in Table VI-D below, emulsion compositions containing a N-alkylphthalamate broke substantially quicker than various commercial products tested.

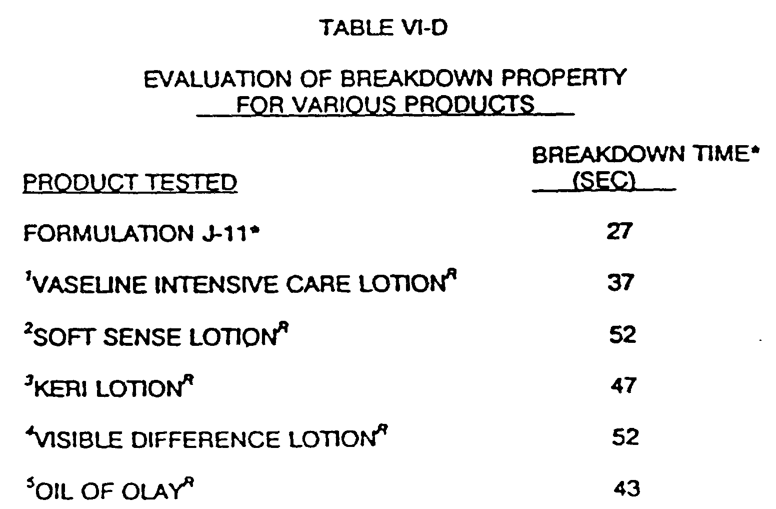

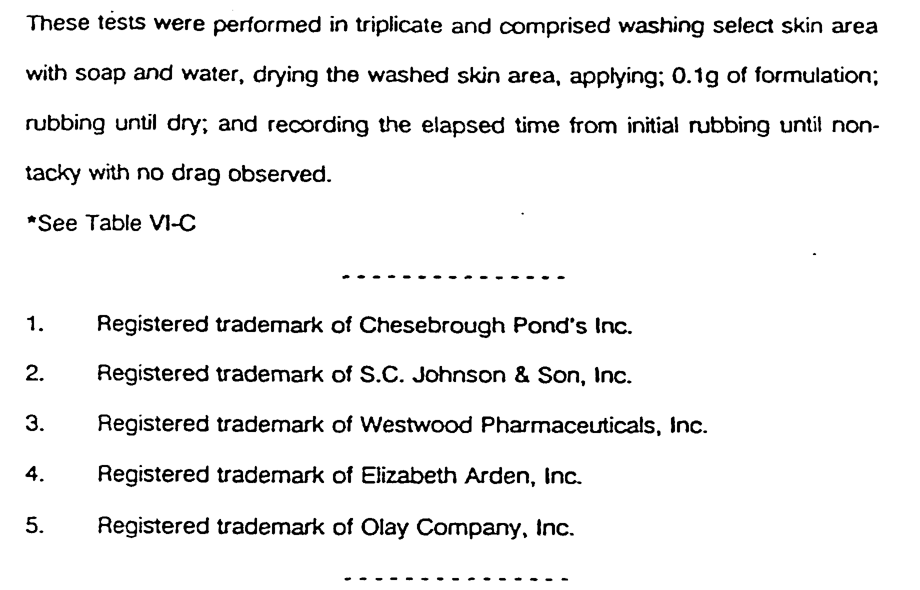

Personal care product compositions (such as skin-care lotions or creams) containing the surfactants of the invention exhibit excellent salt tolerance (i.e., up to about 3% of sodium chloride can be incorporated into select compositions without breaking the emulsions).

Further, typical emulsion compositions exhibit excellent solvent tolerance. Stabile emulsion compositions having up to 20% SD-40 alcohol and/or SD-3A alcohol have been successfully prepared.

The N-alkylphthalamate surfactants of the invention can be incorporated into personal-care product compositions via an oil phase or a water phase. Final emulsions are preferably prepared using a normal two phase hot process.

A wide variety of personal-care emollients, such as petrolatum light, mineral oil, isopropyl palmitate, caprylic/capric triglyceride, avacado oil, sesame seed oil, safflower oil, corn oil, peanut oil, lanolin AAA deodorized, acetylated lanolin, melanin, a variety of sunscreens and vitamins, etc., are readily emulsified by a combination of the relatively high HLB emulsifiers of the invention with a relatively low HLB emulsifier, such as glycerol mono-stearate and a cosmetic emollient of choice so as to attain a ratio of emollient to emulsifier in the range of about 3:1 to about 30:1.

### EXAMPLE D

### Industrial Oily Materials Emulsification Characteristics

Certain industrial oily materials, such as linseed oil, tall oil, soybean oil alkyd, linseed oil alkyd, various cuts of distilled petroleum oil, heavy paraffinic oil, asphaltene oil, etc., are useful in a wide variety of applications, such as in road construction, as resin binders, etc., but are extremely difficult to manipulate due to their inherent viscosity, adhesiveness, lack of spreadibility, etc. Attempts to emulsify these oily materials have met with only limited success and typically involved the use of a plurality of high HLB and low HLB emulsifiers and non-aqueous solvents which, at best, form non-stabile emulsions.

For example, linseed oil is used in road constructions as a binding material prior to repairing at least certain roadways. Linseed oil is an extremely sticky and heavy material and is very difficult to apply in a somewhat uniform manner. Heretofore, linseed oil, per se, or in combination with non-aqueous solvents, was loaded onto a specialized truck having a heated tank maintained at a fairly high temperature (i.e, about 90° to 100°C). The tank truck included feed pipes leading to spray nozzles extended along a manifold to cover a given surface area (i.e., a road lane). The heated linseed oil was pumped through the nozzles, which in some instances were also connected to separate emulsion/solvent solution storage tanks so as to briefly form an emulsion at the nozzle head. This emulsion was then sprayed onto the work surface for manual spreading. Once the linseed oil cooled down and/or the emulsion broke, the linseed oil became very difficult to spread uniformly with manual spreading tools. As will be appreciated, with this type of system, the spray nozzles tend to dog-up and are difficult to dean and workers are exposed to organic vapors and excessive heat.

In accordance with the principles of the invention, linseed oil was readily emulsified into an aqueous system utilizing the phthalamate surfactants (a relatively high HLB emulsifier) in combination with a relatively low HLB emulsifier, such as glycerol stearates, preferably glycerol monostearate, and water. The resulting emulsions were very easy to spray and spread to a desired area. Emulsified linseed oil was stored over extended periods of time without noticeable separation. Further, emulsified linseed oil does not require high temperature for application, thereby providing an economical as well as an environmental advantage.

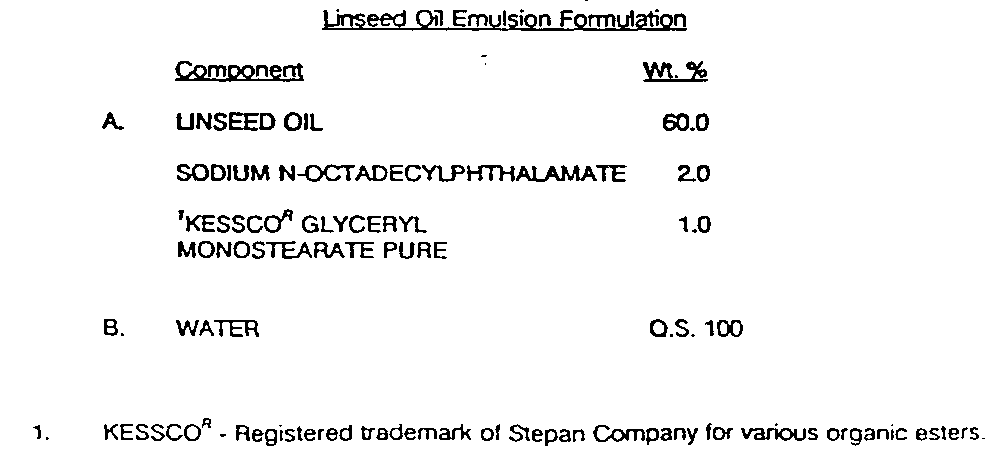

Components A, above, were charged into a heatable vessel and heated with agitation to a temperature of about 77° to 80°C. Component B, above, was charged into a separate heatable vessel and also heated to about 77° to 80°C. Thereafter, component A was added to component B with agitation and continued heating for about 30 minutes or until the emulsion formed. Thereafter, agitation was continued and the emulsion was allowed to cool to about 32°C. Agitation was stopped and emulsion transferred for storage and evaluation. Emulsion stability was evaluated at about 43°C for a 1 month period, at room temperature for a 3 month period and at about 2°C for a 1 month period. In each instance, the emulsion was stabile and was easily applied onto glass slides.

Similarly, other industrial oily materials such as polybutylenes, polyethylenes silicones, polysilicones, gums, etc., can readily be emulsified by combining the relatively high HLB phthalamate emulsifiers of the invention with a relatively low HLB emulsifier, up to about 80 percent by weight of an oily material and Q.S. 100 water.

As is apparent from the foregoing specification, the invention is susceptible of being embodied with various alterations and modifications which may differ particularly from those that have been described in the preceding specification and description. For this reason it is to be fully understood that all the foregoing is intended to be merely illustrative. It is not to be construed or interpreted as being restrictive or otherwise limiting of the present invention, excepting as it is set forth and defined in the hereto-appended claims.

## Claims

1. A composition maintained as an emulsion of an oil phase and a water phase, characterized in that the emulsion comprises an emulsifier of the formula: 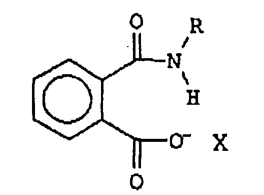
where R represents C₁₆-C₁₈ alkyl;
X represents a sodium or triethanolammonium ion; and the composition is at a pH of 7 to 10.

2. A composition according to Claim 1, where X is triethanolammonium and R represents C₁₈ alkyl.

3. A composition according to Claim 1, where X is a sodium ion and R represents C₁₈ alkyl.

4. A composition according to Claim 1, where X is a sodium ion and R represents C₁₆ alkyl,

5. A composition according to any of the preceding claims further comprising a glyceryl mono-fatty acid ester.

6. A composition according to any of the preceding claims where the composition comprises at least about 0.5% by weight of the emulsifier.

7. A composition according to any of the preceding claims where the composition is a fast-breaking emulsion having a breakdown time of less than 30 seconds.

8. A composition according to any of claims 1, 5, 6 and 7, where the emulsifier is sodium N-octadecyl phthalamate.

9. A composition according to any of the preciding claims, where the weight ratio of the oil phase to the emulsifier being is at least 6.67:1.

10. A composition according to any of the preciding claims, where the oil phase comprises mineral oil, isopropyl myristate, aloe vera oil, glycerine, caprylic/capric triglyceride, petrolatum light, avocado oil, sesame seed oil, safflower oil, corn oil, peanut oil, lanolin, acetylated lanolin, melanin, linseed oil, polybutylene, polyethylene, silicone, polysilicone, gum or mixtures thereof.

## Patentansprüche

1. Zusammensetzung, die als Emulsion einer Ölphase und einer Wasserphase aufrechterhalten ist, dadurch gekennzeichnet, daß die Emulsion einen Emulgator der Formel 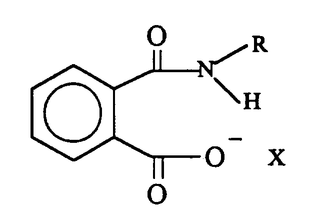 aufweist, in der
R für C₁₆-C₁₈-Alkyl steht;
X für ein Natrium- oder ein Triethanolammoniumion steht; und daß die Zusammensetzung einen pH von 7 bis 10 aufweist.

2. Zusammensetzung nach Anspruch 1, bei der X Triethanolammonium ist und R für C₁₈-Alkyl steht.

3. Zusammensetzung nach Anspruch 1, bei der X ein Natriumion ist und R für C₁₈-Alkyl steht.

4. Zusammensetzung nach Anspruch 1, bei der X ein Natriumion ist und R für C₁₆-Alkyl steht.

5. Zusammensetzung nach einem der vorangegangenen Ansprüche, die außerdem einen Glyceryl-Monofettsäureester enthält.

6. Zusammensetzung nach einem der vorangegangenen Ansprüche, bei der die Zusammensetzung zumindest etwa 0,5 Gew.-% des Emulgators aufweist.

7. Zusammensetzung nach einem der vorangegangenen Ansprüche, bei der die Zusammensetzung eine schnell brechende Emulsion ist, die eine Aufspaltzeit von weniger als 30 Sekunden aufweist.

8. Zusammensetzung nach einem der Ansprüche 1, 5, 6 oder 7, bei der der Emulgator Natrium-N-octadekylphthalamat ist.

9. Zusammensetzung nach einem der vorangegangenen Ansprüche, bei der das Gewichtsverhältnis von Ölphase zu Emulgator zumindest 6,67 : 1 beträgt.

10. Zusammensetzung nach einem der vorangegangenen Ansprüche, bei der die Ölphase Mineralöl, Isopropylmyristat, Aloeveraöl, Glycerin, Caprylsäure-/Caprinsäure-Triglyceride, Petrolatum leicht, Avocadoöl, Sesamsamenöl, Safloröl, Maisöl, Erdnußöl, Lanolin, acetyliertes Lanolin, Melanin, Leinöl, Polybutylen, Polyethylen, Silkon, Polysilikon, Gum oder Mischungen derselben enthält.

## Revendications

1. Composition sous la forme d'une émulsion d'une phase huileuse et d'une phase aqueuse, caractérisée en ce que l'émulsion comprend un émulsifiant répondant à la formule suivante : 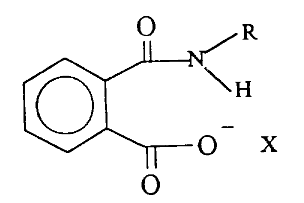
dans laquelle
R représente un alkyle en C₁₆-C₁₈ ;
X représente l'ion sodium ou triéthylammonium ; et en ce que la composition a un pH allant de 7 à 10.

2. Composition selon la revendication 1, dans laquelle X représente l'ion triéthylammonium et R représente un alkyle en C₁₈.

3. Composition selon la revendication 1, dans laquelle X représente l'ion sodium et R représente un alkyle en C₁₈.

4. Composition selon la revendication 1, dans laquelle X représente l'ion sodium et R représente un alkyle en C₁₆.

5. Composition selon l'une quelconque des revendications précédentes, comprenant en outre un monoester d'acide gras et de glycérol.

6. Composition selon l'une quelconque des revendications précédentes, comprenant l'émulsifiant à une concentration d'au moins environ 0,5% en poids.

7. Composition selon l'une quelconque des revendications précédentes, consistant en une émulsion à rupture rapide présentant un temps de rupture inférieur à 30 secondes.

8. Composition selon l'une quelconque des revendications 1, 5, 6 et 7, dans laquelle l'émulsifiant est le N-octadécyl-phtalamate de sodium.

9. Composition selon l'une quelconque des revendications précédentes, dans laquelle le rapport du poids de la phase huileuse au poids de l'émulsifiant est au moins égal à 6,67:1.

10. Composition selon l'une quelconque des revendications précédentes, dans laquelle la phase huileuse comprend une huile minérale, du myristate d'isopropyle, de l'huile d'aloes vera, de la glycérine, le triglycéride caprylique / caprique, de la vaseline légère, de l'huile d'avocat, de l'huile de graine de sésame, de l'huile de carthame, de l'huile de maïs, de l'huile d'arachide, de la lanoline, de la lanoline acétylée, de la mélanine, de l'huile de graine de lin, un polybutylène, un polyéthylène, un silicone, un polysilicone, une gomme, ou un mélange de ceux-ci.
